# EUROPÄISCHE PATENTSCHRIFT

(11) **EP 2 452 703 B1**
(45) Veröffentlichungstag und Bekanntmachung des Hinweises auf die Patenterteilung: **04.03.2015**
(21) Anmeldenummer: 11188034.0
(22) Anmeldetag: 07.11.2011
(51) Int. Cl.: A61K 47/48, C07C 33/00, C08B 37/08, A61L 31/10, A61L 31/16

(54) **Funktionalisierte RGD-Peptidmimetika und deren Herstellung sowie Implantat mit einer Beschichtung, die solche funktionalisierte RGD-Peptidmimetika enthält**
Functionalized RGD peptidomimetics and their manufacture, and implant having a coating containing such functionalized RGD peptidomimetics
Mimétiques de peptides RGD fonctionnalisées et leur fabrication ainsi qu'implant doté d'un revêtement, lequel comprend de telles mimétiques de peptide RGD fonctionnalisées

(30) Priorität: 12.11.2010 US 412804 P
(43) Veröffentlichungstag der Anmeldung: 16.05.2012
(73) Patentinhaber: Biotronik AG, 8180 Bülach (CH)
(72) Erfinder: Borck, Alexander, 91086 Aurachtal (DE); Gratz, Matthias, 91054 Erlangen (DE); Kessler, Horst, 85748 Garching (DE); Joner, Michael, 82178 Puchheim (DE); Rechenmacher, Florian, 80939 München (DE); Neubauer, Stefanie, 80939 München (DE)
(74) Vertreter: Lindner-Vogt, Karin L.

(56) Entgegenhaltungen:
- EP-A1- 2 055 328
- WO-A2-00/49135
- US-A1- 2007 280 991
- CLAUDIA DAHMEN ET AL: "Verbesserte Implantatmaterialien durch die Beschichtung mit hochspezifischen, nichtpeptidischen Integrinliganden", ANGEWANDTE CHEMIE (INTERNATIONAL ED. IN ENGLISH), Bd. 116, Nr. 48, 10. Dezember 2004 (2004-12-10), Seiten 6818-6821, XP055134738, ISSN: 0044-8249, DOI: 10.1002/ange.200460770
- SULYOK GABOR A G ET AL: "Solid-phase synthesis of a nonpeptide RGD mimetic library: New selective alphavbeta3 integrin antagonists", JOURNAL OF MEDICINAL CHEMISTRY, AMERICAN CHEMICAL SOCIETY, US, Bd. 44, Nr. 12, 7. Juni 2001 (2001-06-07), Seiten 1938-1950, XP002175054, ISSN: 0022-2623, DOI: 10.1021/JM0004953

## Beschreibung

Die Erfindung betrifft funktionalisierte RGD-Peptidmimetika und ein dazugehöriges Herstellungsverfahren sowie ein Implantat mit einer Beschichtung, die solche RGD-Peptidmimetika enthält.

### Stand der Technik und Hintergrund der Erfindung

Implantate haben in vielfältiger Ausführungsform Anwendung in der modernen Medizintechnik gefunden und umfassen beispielsweise Implantate mit Stützfunktion, z.B. Stents, Implantate mit Steuerungsfunktion, z.B. Elektroden und Implantate mit Mess- oder Überwachungsfunktion, z.B. Sensoren. Implantate können beispielsweise der Unterstützung von Gefäßen, Hohlorganen und Gangsystemen (Endovaskuläre Implantate, z. B. Stents), zur Befestigung und temporären Fixierung von Gewebeimplantaten und Gewebstransplantationen dienen, aber auch zu orthopädischen Zwecken, zum Beispiel als Nagel, Platte oder Schraube. Eine besonders häufig verwendete Form eines Implantats ist der Stent.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch A-neurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Das Implantat, insbesondere der Stent, besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiA16V4 oder Ti-A16Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken, und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem beispielsweise der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Bekannt ist, dass sich ein höheres Maß an Biokompatibilität erreichen lässt, wenn Implantatwerkstoffe mit Beschichtungen aus besonders gewebsverträglichen Materialien versehen werden. Diese Materialien sind zumeist organischer oder synthetisch-polymerer Natur und teils natürlichen Ursprungs. Weitere Strategien zur Vermeidung der Restenose konzentrieren sich darauf, die Proliferation durch Medikation, z. B. Behandlung mit Cytostatika, zu hemmen. Die Wirkstoffe können zum Beispiel auf der Implantatoberfläche in Form einer Wirkstoff-freisetzenden Beschichtung bereitgestellt werden.

Es ist ferner bekannt, dass die Triade RGD (Arg-Gly-Asp) vielen Integrinen als eine primäre Erkennungssequenz für Proteine der extrazellulären Matrix dient. Peptide, welche diese Sequenz enthalten, können somit die Liganden dieser Integrine nachahmen und daher an diese binden.

Aufgrund der Tatsache, dass RGD-Peptide selektive Antagonisten für Integrine sind, werden sie bzw. von ihnen abgeleitete Peptidmimetika hinsichtlich ihrer medizinischen Relevanz erforscht.

WO 00/49135 beschreib Substrate für Zellwachstum, z.B. Gefäßtransplantate oder orthopädische Implantate. Die Substrate umfassen auf ihrer Oberfläche eine Basisschicht von z.B. Chitosan oder Polylysine, und ein Zelladhäsionsprotein. Das Zelladhäsionsprotein umfasst vorzugsweise eine RGD-Bindungsstelle. Das Zelladhäsionsprotein kann kovalent an die Basisschicht gekoppelt sein.

So sind zyklische RGD-Peptide (cRGD) oder RGD-Peptidmimetika bereits als Bestandteil einer Implantatbeschichtung vorgeschlagen worden. Es ist bekannt, Polymer-Stents mit Integrin-bindenden cRGD-Peptiden zur Reduktion der neointimalen Hyperplasie durch Attraktion von endothelialen Progenitorzellen zu beladen (Pressemitteilung der Deutschen Gesellschaft für Kardiologie - Herz- und Kreislaufforschung e. V. (DGK), September 2005).

Für die Entwicklung von Implantaten mit Wirkstoff-freisetzender Beschichtung, insbesondere wirkstoffbeschichteter Stents (sogenannter DES- oder "drug eluting"-Stents), stellt der Einsatz von cRGDs oder RGD-Peptidmimetika somit einen vielversprechenden Ansatz zur Verbesserung der Verträglichkeit der Implantate dar. Der Einsatz von RGD-Peptidmimetika ist jedoch vornehmlich zur Verbesserung des Einheilungprozesses notwendig, für langfristige Entwicklungen ist es in der Regel dennoch notwendig, andere Wirkstoffe, z. B. Rapamycin, aus der Beschichtung freizusetzen. Damit besteht bei nachträglicher Aufnahme von RGD-Peptidmimetika in ein bestehendes Beschichtungssystem das Problem, dass zum einen sichergestellt werden muss, dass die RGD-Peptidmimetika zu einem möglichst frühen Zeitpunkt an der Oberfläche des Implantats bereitgestellt werden, sich jedoch andererseits die Elutionscharakteristik eines sich in einer darunter befindlichen Basis der Beschichtung bereitgestellten Wirkstoffs nicht verändern soll. Ansonsten wäre eine erneute Optimierung der Elutionscharakteristik des Wirkstoffs erforderlich, was im Einzelfall sehr aufwendig ist und eine Variation des Systems erschwert.

Es wäre daher von Vorteil, die Bereitstellung von RGD-Peptidmimetika an der Implantatsoberfläche so zu gestalten, dass sich die Elutionscharakteristik aus der darunter liegenden Basisschicht für einen dort eingebetteten Wirkstoff nicht oder nur unwesentlich ändert. Ferner wäre von Vorteil, wenn die Erkennungssequenzen der RGD-Peptidmimetika sofort nach Implantation zugänglich sind, also zum Beispiel nicht erst durch allmähliche Degradation der Beschichtungsmatrix freigelegt werden.

### Zusammenfassung der Erfindung

Die vorliegende Erfindung stellt ein Implantat mit einer Beschichtung bereit, wobei die Beschichtung ein funktionalisiertes RGD-Peptidmimetikum RGD-P1 mit der Formel (1): 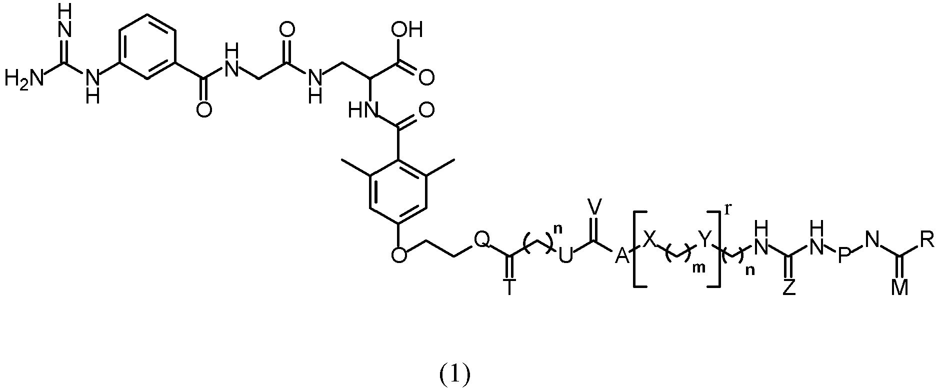 und/oder
ein funktionalisiertes RGD-Peptidmimetikum RGD-P2 mit der Formel (2): 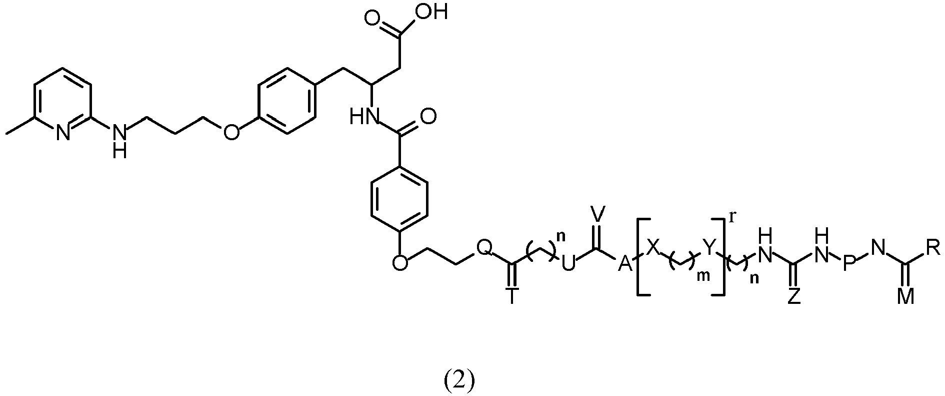 enthält oder daraus besteht.

Dabei steht Q, T sowie U, V unabhängig voneinander für O oder NH, wobei Q≠T und U≠V.

Dabei steht A für O oder (CH₂)₁₋₃ und X für CH₂, O, N oder S, wobei wenn A gleich O ist, X CH₂ sein muss.

Dabei steht Y für CH₂, O, N oder S sowie Z und M unabhängig voneinander für O oder S.

n und r stehen für 1 bis 6 und m steht für 2 bis 8.

Weiterhin steht P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen.

R steht unabhängig voneinander für eine polymere Gruppe, ausgewählt aus Chitosan und Polylysin, wobei Polylysin Poly-D-Lysin und Poly-L-Lysin umfasst, bevorzugtes Polylysin ist dabei Poly-L-Lysin.

Diesem Aspekt der Erfindung liegt demnach unter anderem die Erkenntnis zugrunde, dass sich mit Hilfe der erfindungsgemäßen Kopplung an die Polymere Chitosan oder Polylysin die erhaltenen funktionalisierten RGD-Peptidmimetika in besonders vorteilhafter Weise als dünne Schicht auf ein Implantat auftragen lassen und dabei sowohl ihre Integrinspezifität als auch ihre Integrinbindungseigenschaften im Wesentlichen erhalten bleiben. Weist ein Implantat auf einer Wirkstoff-freisetzenden Basisbeschichtung eine Schicht enthaltend oder bestehend aus den funktionalisierten RGD-Peptidmimetika RGD-P1 gemäß Formel (1) und/oder RGD-P2 gemäß der Formel (2) auf, beispielsweise in Form einer Deckschicht, so wird die Elutionscharakteristik der darunter liegenden Basis für den Wirkstoff nicht oder allenfalls nur in sehr geringem Umfang verändert. Das erfindungsgemäße Implantat zeichnet sich also dadurch aus, dass das Einwachsen des Implantats durch die Integrinbindung der funktionalisierten RGD-Peptidmimetika gefördert wird, während sich keine oder nur vernachlässigbare Effekte auf die Eigenschaften von darunter liegenden Schichten ergeben.

Unter einem RGD-Peptidmimetikum wird eine chemische Verbindung verstanden, die sich bezüglich der Bindungseigenschaften an ausgewählte Integrine im Wesentlichen verhält wie das zugrunde liegende Protein, sich aber strukturell von dem zugrunde liegenden Protein unterscheidet. In der Regel weisen Peptidmimetika kein durchgängiges Peptidrückgrat aus Peptidbindungen auf und sind meist nicht ausschließlich aus proteinogenen Aminosäuren aufgebaut.

Im vorliegenden Fall handelt es sich bei dem funktionalisierten RGD-Peptidmimetikum RGD-P1 mit der Formel (1) um eine Verbindung, die eine Integrinbindungsdomäne aufweist, die bevorzugt an a₅ß₁ Integrin bindet. Die Integrinbindungsdomäne des RGD-P1 umfasst unter Anderem folgende Struktur:

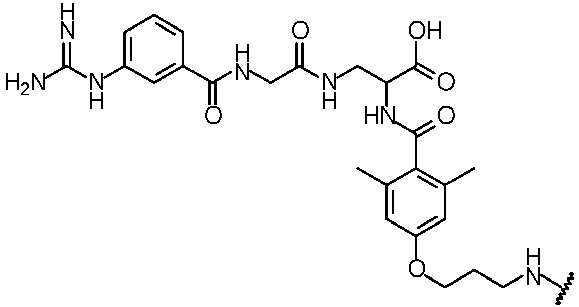

Im Fall des funktionalisierten RGD-Peptidmimetikums RGD-P2 mit der Formel (2) handelt es sich um eine Verbindung, die eine Integrinbindungsdomäne aufweist, die bevorzugt an aᵥß₃ Integrin bindet. Die Integrinbindungsdomäne des RGD-P2 umfasst unter Anderem folgende Struktur:

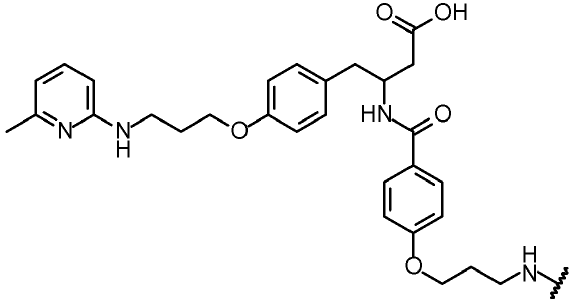

An die Integrinbindungsdomänen der RGD-Peptidmimetika RGD-P1 und RGD-P2 schließt sich jeweils eine Aminoalkansäure an, die als Abstandshalter (sog. "spacer") eingesetzt wird.

Die RGD-Peptidmimetika RGD-P1 und RGD-P2 sind an der freien Aminogruppe der Aminoalkansäure derart funktionalisiert, dass sie wiederum über eine der freien Aminogruppen der Polymere Chitosan oder Polylysin kovalent verbunden sind.

Das erhaltene funktionalisierte RGD-Peptidmimetikum stellt also ein Polymer dar auf Basis des Chitosan oder Polylysins, wobei an einer, mehrerer oder allen freien Aminogruppen des Chitosans oder des Polylysins über die Kopplungsgruppe Isocyanat oder Thioisocyanat und eine Aminoalkansäure eine spezifische RGD-basierte Integrinbindungsdomäne gekoppelt ist.

Um nach erfolgter Implantation einen möglichst direkten Kontakt der Integrinbindungsdomänen der RGD-Peptidmimetika des Implantats mit Integrinen von umgebenden Zellen zu erlauben, weist das erfindungsgemäße Implantat die Beschichtung enthaltend das funktionalisierte RGD-Peptidmimetikum RGD-P1 gemäß Formel (1) und/oder RGD-P2 gemäß Formel (2) als Deckschicht auf. Unter einer Deckschicht wird dabei eine Schicht verstanden, die das Implantat mindestens abschnittweise als oberste Beschichtung gegen die Umwelt abgrenzt. In einer bevorzugten Ausführungsform schließt die Deckschicht die Beschichtung des Implantats auf der dem Grundkörper des Implantats abgewandten Seite ab.

Unter der Deckschicht kann das Implantat eine Basisschicht aufweisen, die ggf. aus mehreren unterschiedlichen Schichten bestehen kann. Die Deckschicht bedeckt die Basisschicht dabei mindestens teilweise oder ganz. Die Basisschicht kann eine Wirkstoff-freisetzende Schicht umfassen.

Eine Beschichtung im Sinne der Erfindung ist eine zumindest abschnittsweise Auftragung der Komponenten der Beschichtung auf den Grundkörper des Implantats. Vorzugsweise wird die gesamte Oberfläche des Grundkörpers des Implantats von der Beschichtung bedeckt. Eine Schichtdicke liegt vorzugsweise im Bereich von 1 nm bis 100 µm, besonders bevorzugt 300 nm bis 15 µm. Die Beschichtung kann direkt auf die Implantatoberfläche aufgetragen werden. Die Verarbeitung kann nach Standardverfahren für die Beschichtung erfolgen. Als Basisschicht können einschichtige, aber auch mehrschichtige Systeme (zum Beispiel so genannte base coat -, drug coat - oder top coat - Schichten) erstellt werden. Die Basisschicht kann unmittelbar auf dem Grundkörper des Implantats aufgebracht sein oder es sind weitere Schichten dazwischen vorgesehen. Verfahren zur Beschichtung von Implantaten sowohl zur Erstellung der Basisschicht als auch zur Erstellung der Deckschicht sind dem Fachmann bekannt.

Unter Wirkstoff im erfindungsgemäßen Sinne wird ein Arzneistoff mit pharmazeutischer Wirkung verstanden, der im menschlichen oder tierischen Körper zur Heilung, Linderung, Verhütung oder Erkennung von Krankheiten dient. Wirkstoffe umfassen insbesondere Paclitaxel, Sirolimus, Rapamycin und Rapamycinderivate. Insbesondere Wirkstoffe, die über die mTOR-Erkennungssequenz wirken. Ferner RAS-Inhibitoren, insbesondere solche, die die RAS-Adhäsion an die Zellmembran verhindern.

Bevorzugt handelt es sich bei dem erfindungsgemäßen Implantat um einen Stent, besonders bevorzugt um einen biokorrodierbaren Stent. Dazu kann der Stent einen Grundkörper aufweisen, der einen biodegradierbaren Implantatwerkstoff enthält oder daraus besteht. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen. Insbesondere kann der Grundkörper des erfindungsgemäßen Stents eine biokorrodierbare Magnesiumlegierung aufweisen oder daraus bestehen.

Unter Legierung wird vorliegend ein metallisches Gefüge verstanden, deren Hauptkomponente Magnesium, Eisen, Zink oder Wolfram ist. Hauptkomponente ist die Legierungskomponente, deren Gewichtsanteil an der Legierung am höchsten ist. Ein Anteil der Hauptkomponente beträgt vorzugsweise mehr 50 Gew.%, insbesondere mehr als 70 Gew.%.

Die Legierungen der Elemente Magnesium, Eisen, Zink oder Wolfram können so in ihrer Zusammensetzung gewählt sein, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

In DE 197 31 021 A1 werden geeignete biokorrodierbare metallische Implantatwerkstoffe vorgestellt, deren Hauptbestandteil ein Element aus der Gruppe Alkalimetalle, Erdalkalimetalle, Eisen, Zink und Aluminium ist. Als besonders geeignet werden Legierungen auf Basis von Magnesium, Eisen und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung eines Stents, z. B. in Form eines selbstexpandierenden oder ballonexpandierbaren Stent, eignet.

Ein weiterer Aspekt der Erfindung liegt in der Bereitstellung eines Verfahrens zur Herstellung eines obig genannten Implantates. Das Verfahren umfasst die Schritte:
- Beschichtung eines Stents mit einer Schicht, die Chitosan und/oder Polylysin enthält oder daraus besteht;
- kovalente Kopplung eines in Chitosan und/oder Polylysin enthaltenen primären Amins mit einem Vorläufer des RGD-Peptidmimetikums RGD-P1 mit der Formel (3): 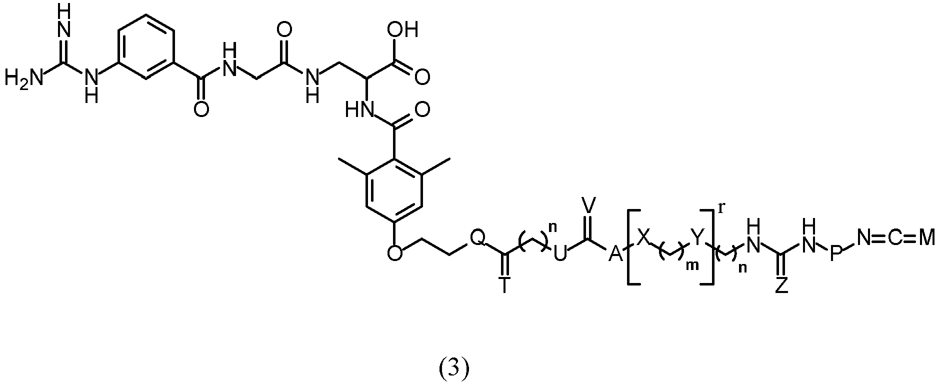 und/oder
   eines Vorläufers des RGD-Peptidmimetikums RGD-P2 mit der Formel (4):

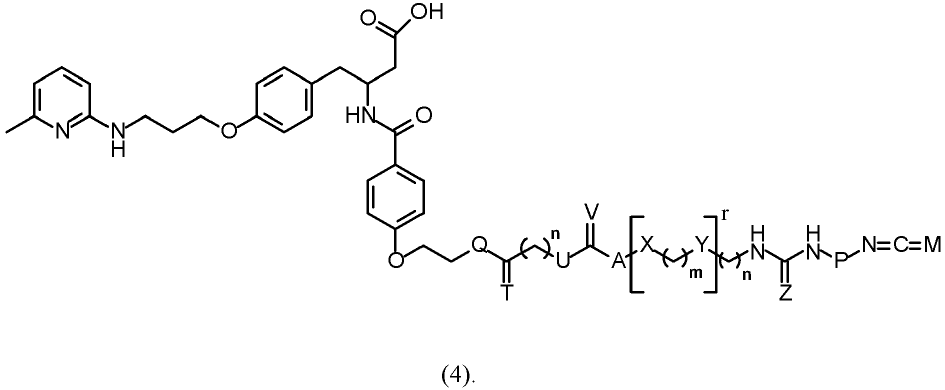

Dabei steht Q, T sowie U, V unabhängig voneinander für O oder NH, wobei Q≠T und U≠V.

Dabei steht A für O oder (CH₂)₁₋₃ und X für CH₂, O, N oder S, wobei wenn A gleich O ist, X CH₂ sein muss.

Dabei steht Y für CH₂, O, N oder S sowie Z und M unabhängig voneinander für O oder S.

n und r stehen für 1 bis 6 und m steht für 2 bis 8.

Weiterhin steht P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen.

Mit dem vorgeschlagenen Verfahren können beispielsweise auch bereits bestehende Wirkstoff-freisetzende Implantate, Stents oder Systeme im Anschluss an die eigentliche Wirkstoffbeschichtung mit einer Deckschicht enthaltend die funktionalisierten RGD-Peptidmimetika RGD-P1 und/oder RGD-P2 beschichtet werden.

Ein besonderer Vorteil hierbei ist, dass die Wirkstoffe, die bereits auf der Oberfläche des Wirkstoff-freisetzende Implantats beschichtet vorliegen, unter Erhalt ihrer biologischen Aktivität mit der Beschichtung enthaltend die erfindungsgemäßen Verbindungen versehen werden können. Die meisten dem Fachmann bekannte Methoden zum beschichten eines vorab mit bspw. Sirolimus-beschichteten Stents führen zu einer teilweisen Zersetzung des Wirkstoffs, was zu einer verminderten biologischen Aktivität des Implantats führt.

Die Beschichtung des erfindungsgemäßen Implantats mit einem funktionalisierten RGD-Peptidmimetikum kann folgendermaßen hergestellt werden. Chitosan oder Polylysin kann in verdünnter wässriger Lösung zum Beispiel auf einen ggf. auch mit einem wirkstoffbeladenen Basismaterial vorbeschichteten Stent durch einen Sprüh- oder Tauchprozess aufgebracht werden. Die nachträgliche Ankopplung von Vorläufern der RGD-Peptidmimetika RGD-P1 und/oder RGD-P2 geschieht dann im wässrigen Medium, in dem bei lipophilen Wirkstoffen nicht die Gefahr besteht, dass in diesem Medium die in der Basis eingebundenen Wirkstoffe eluieren und damit die Wirkstoffbeladung sinkt.

In einem weiteren Aspekt stellt die vorliegende Erfindung Verbindungen bereit gemäß der:
Formel (1) 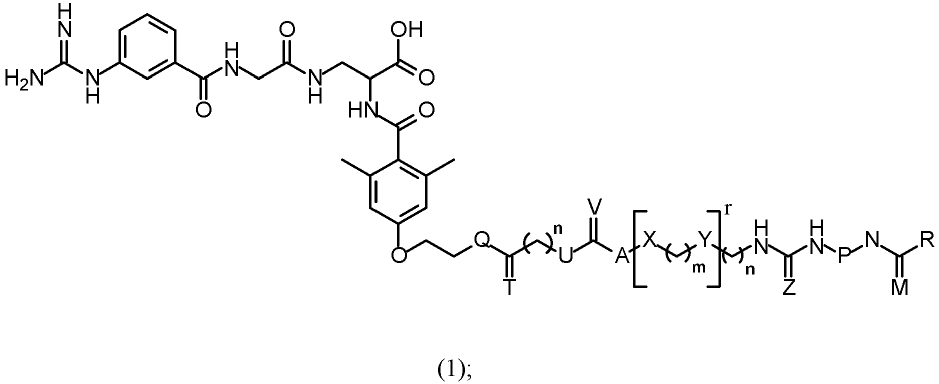
Formel (2) 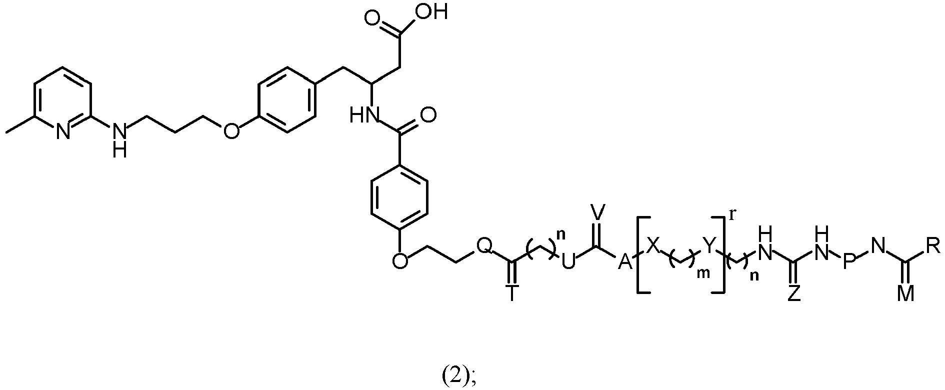
Formel (3) 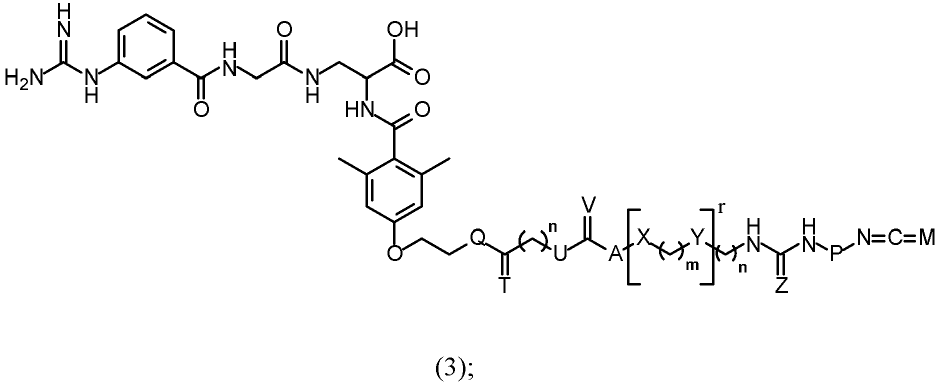
oder Formel (4)

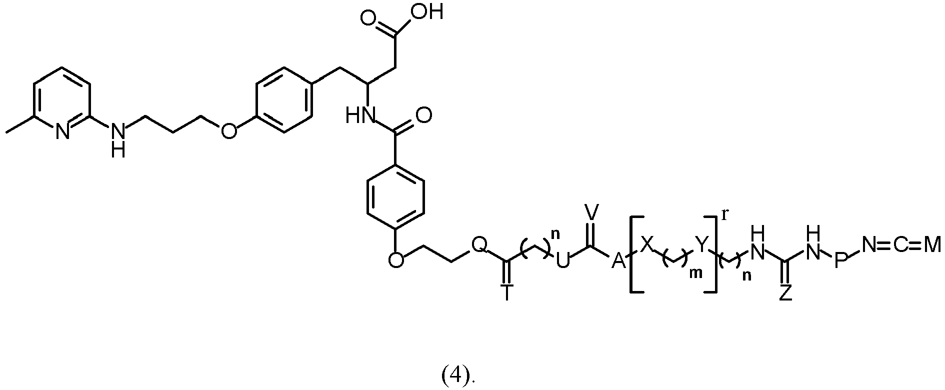

Dabei steht Q, T sowie U, V unabhängig voneinander für O oder NH, wobei Q≠T und U≠V.

Dabei steht A für O oder (CH₂)₁₋₃ und X für CH₂, O, N oder S, wobei wenn A gleich O ist, X CH₂ sein muss.

Dabei steht Y für CH₂, O, N oder S sowie Z und M unabhängig voneinander für O oder S.

n und r stehen für 1 bis 6 und m steht für 2 bis 8.

Weiterhin steht P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen.

R steht unabhängig voneinander für eine polymere Gruppe, ausgewählt aus Chitosan und Polylysin, wobei Polylysin Poly-D-Lysin und Poly-L-Lysin umfasst, bevorzugtes Polylysin ist dabei Poly-L-Lysin.

Die genannten erfindungsgemäßen Verbindungen können insbesondere in einer Beschichtung eines Implantates, insbesondere in der Deckschicht eines Wirkstoff-freisetzenden Stents verwendet werden, wobei der Stent einen resorbierbaren oder permanenten Grundkörper aus einem metallischen oder polymeren Material aufweist.

Die Erfindung wird nachfolgend anhand von Ausführungsbeispielen näher erläutert.

### Umsetzung der Verbindung der Formel (3) und (4) an einer Chitosan-Oberfläche

Ein Stent mit einer Beschichtung aus Polylactid (PLLA) und Sirolimus wird mit einer Lösung von Chitosan in verdünnter Essigsäure (0,3 %) besprüht. Nach dem Trocknen wird der Stent in eine gepufferte (Phosphatpuffer 50mM) wässrige Lösung eines Vorläufers des funktionalisierten RGD-Peptidmimetikums RGD-P1 mit der Formel (3) oder eines Vorläufers des funktionalisierten RGD-Peptidmimetikums RGD-P2 mit der Formel (4) getaucht (Konzentration: 5 mg/ml; pH 7; Raumtemperatur). Nach 1h sind alle Amingruppen des Chitosan mit den reaktiven Gruppen der Kopplungsgruppen abreagiert.

Der Stent zeigt keine abweichende Elutionskinetik für Sirolimus. Im Gegensatz zu rein mit PLLA und Sirolimus beschichteten Stents ist schon 3 Tage nach Implantation im Tier eine erste Endothelbedeckung sichtbar.

## Patentansprüche

1. Implantat mit einer Beschichtung, wobei die Beschichtung ein funktionalisiertes RGD-Peptidmimetikum RGD-P1 mit der Formel (1): 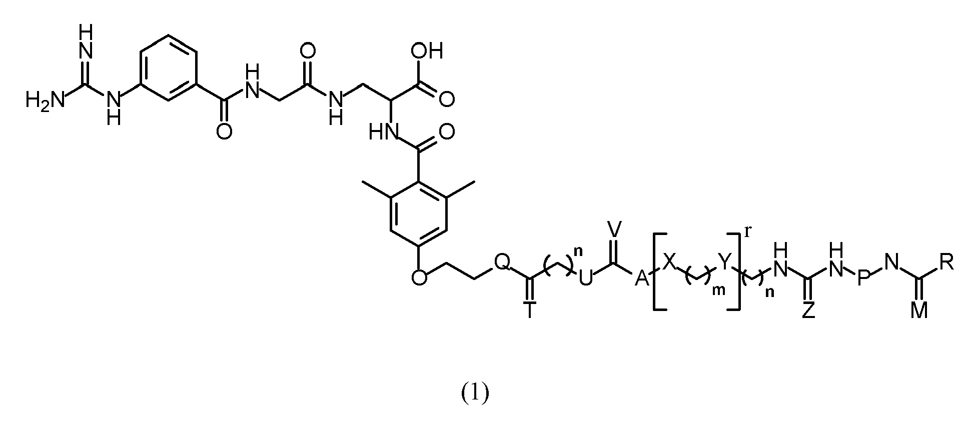 und/oder
ein funktionalisiertes RGD-Peptidmimetikum RGD-P2 mit der Formel (2): 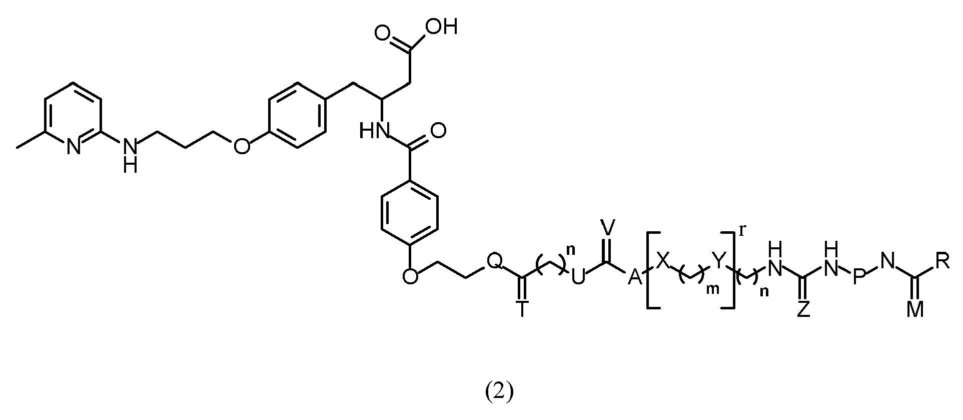 enthält oder daraus besteht, wobei
Q, T sowie U, V unabhängig voneinander für O oder NH steht und Q≠T und U≠V;
A für O oder (CH₂)₁₋₃ und X für CH₂, O, N oder S steht und wenn A gleich O ist, X CH₂ ist;
Y für CH₂, O, N oder S sowie Z und M unabhängig voneinander für O oder S steht; n und r für 1 bis 6 stehen und m für 2 bis 8 steht;
P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen steht; und
R unabhängig voneinander für eine polymere Gruppe ausgewählt aus Chitosan und Polylysin steht, wobei Polylysin Poly-D-Lysin und Poly-L-Lysin umfasst.

2. Implantat nach Anspruch 1, wobei die Beschichtung enthaltend das funktionalisierte RGD-Peptidmimetikum RGD-P1 der Formel (1) und/oder RGD-P2 der Formel (2) als Deckschicht ausgebildet ist.

3. Implantat nach Anspruch 1 oder 2, wobei die Beschichtung des Implantats eine Basisschicht umfasst und eine auf der Basisschicht aufgetragene Deckschicht enthaltend das funktionalisierte RGD-Peptidmimetikum RGD-P1 der Formel (1) und/oder RGD-P2 der Formel (2).

4. Implantat nach Anspruch 3, wobei die Basisschicht eine Wirkstoff-freisetzende Schicht aufweist.

5. Implantat nach Anspruch 4, wobei der Wirkstoff Paclitaxel, Sirolimus und/oder Rapamycin ist, bevorzugt ist der Wirkstoff Sirolimus.

6. Implantat nach einem der Ansprüche 1 bis 5, wobei das Implantat ein Stent, ein Sensor oder eine Elektrode ist.

7. Implantat nach Anspruch 6, wobei der Stent einen resorbierbaren oder permanenten Grundkörper aus einem metallischen oder polymeren Material aufweist.

8. Verfahren zur Herstellung eines Implantats nach einem der Ansprüche 1 bis 7, wobei das Verfahren die Schritte umfasst:
- Beschichtung eines Stents mit einer Schicht, die Chitosan und/oder Polylysin enthält oder daraus besteht;
- kovalente Kopplung eines in Chitosan und/oder Polylysin enthaltenen primären Amins mit der Kopplungsgruppe eines Vorläufers des funktionalisierten RGD-Peptidmimetikums RGD-P1 mit der Formel (3): 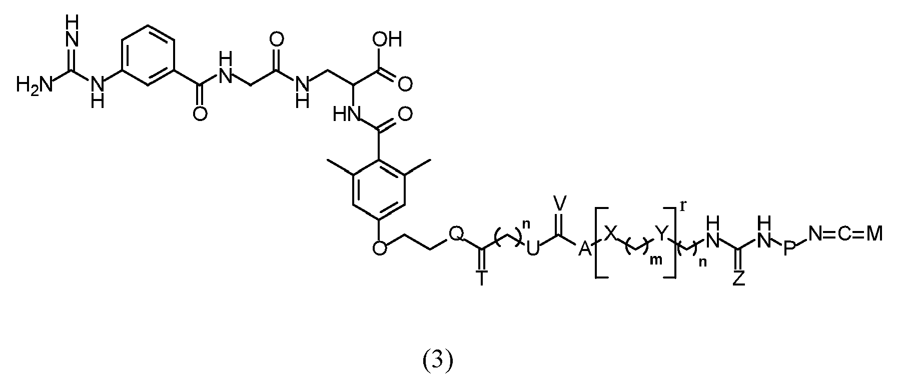 und/oder
eines Vorläufers des funktionalisierten RGD-Peptidmimetikums RGD-P2 mit der Formel (4): 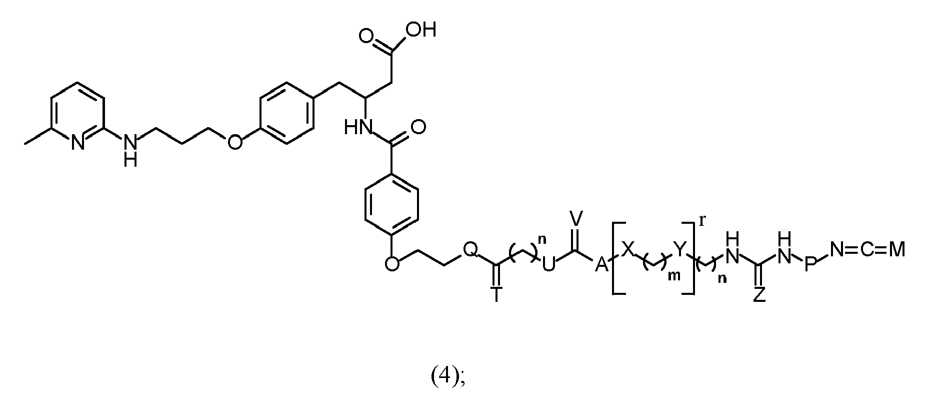 wobei
Q, T sowie U, V unabhängig voneinander für O oder NH steht und Q≠T und U≠V; A für O oder (CH₂)₁₋₃ und X für CH₂, O, N oder S steht und wenn A gleich O ist, X CH₂ ist;
Y für CH₂, O, N oder S sowie Z und M unabhängig voneinander für O oder S steht; n und r für 1 bis 6 stehen und m für 2 bis 8 steht;
P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen steht.

9. Verbindung gemäß:
Formel (1) 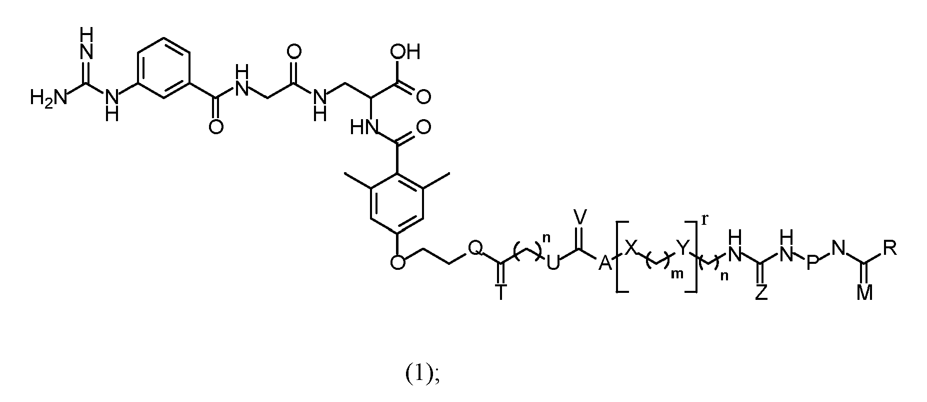
Formel (2) 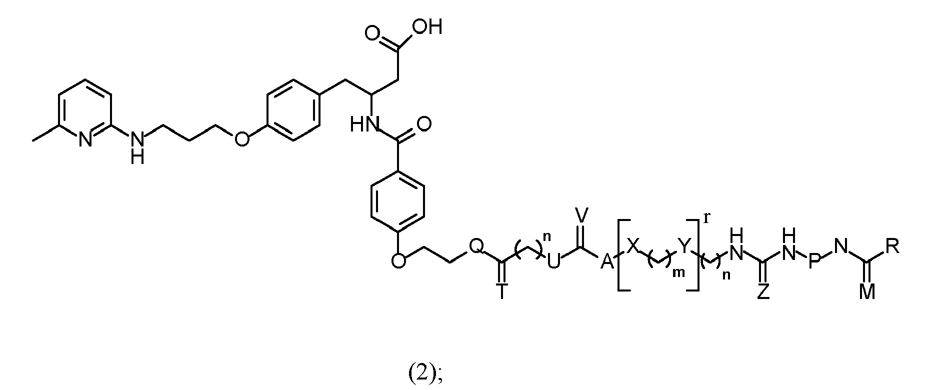
Formel (3) 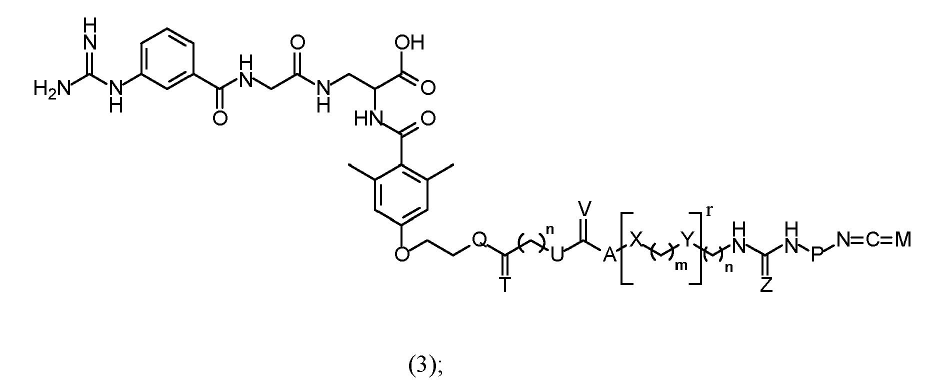 oder
Formel (4) 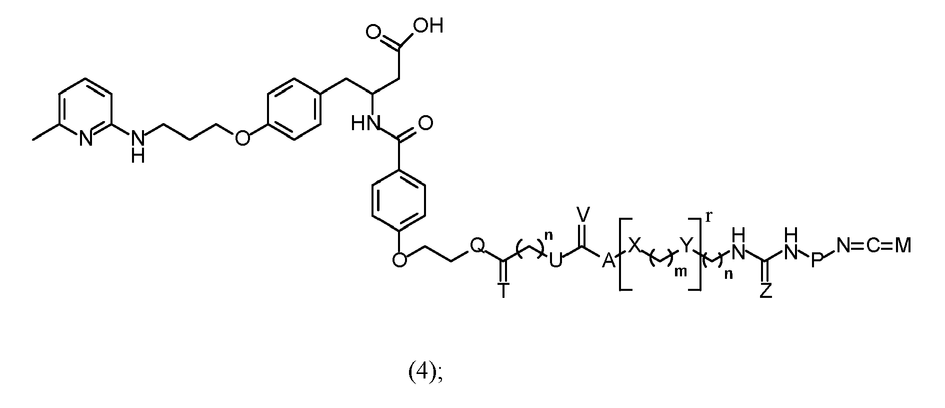 wobei
Q, T sowie U, V unabhängig voneinander für O oder NH steht und Q≠T und U≠V;
A für O oder (CH₂)₁₋₃ und X für CH₂, O, N oder S steht und wenn A gleich O ist, X CH₂ ist;
Y für CH₂, O, N oder S sowie Z und M unabhängig voneinander für O oder S steht; n und r für 1 bis 6 stehen und m für 2 bis 8 steht;
P für eine Gruppe ausgewählt aus substituiertem oder unsubstituiertem Alkylen, bevorzugt (CH₂)₁₋₆, oder substituiertem oder unsubstituiertem, aromatischem oder aliphatischem N- oder O-Cycloalkylen steht; und
R unabhängig voneinander für eine polymere Gruppe ausgewählt aus Chitosan und Polylysin steht, wobei Polylysin Poly-D-Lysin und Poly-L-Lysin umfasst.

10. Verwendung einer Verbindung nach Anspruch 9 in einer Beschichtung eines Implantates, insbesondere in der Deckschicht eines Wirkstoff-freisetzenden Stents.

## Claims

1. An implant having a coating, wherein the coating contains or consists of a functionalised RGD peptidomimetic RGD-P1 having the formula (1): 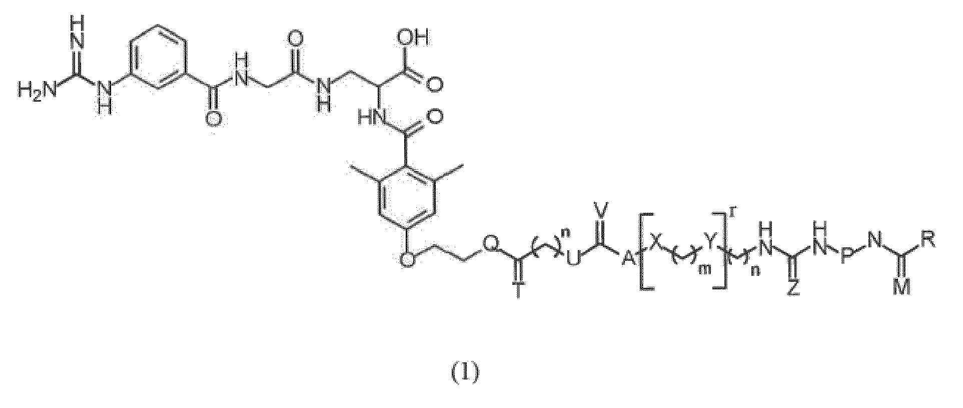 and/or
a functionalised RGD peptidomimetic RGD-P2 having formula (2): 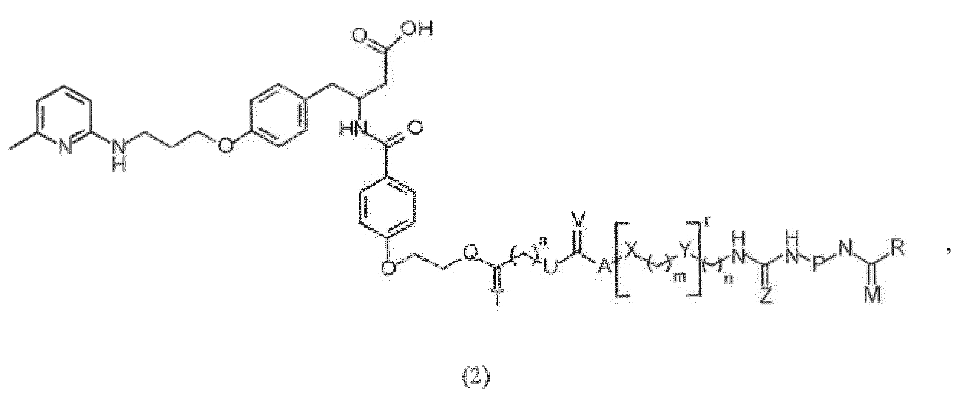 wherein
Q, T and U, V independently of one another stand for O or NH and Q≠T and U≠V;
A stands for O or (CH₂)₁₋₃ and X stands for CH₂, O, N or S, and when A is equal to O, X is CH₂;
Y stands for CH₂, O, N or S, and Z and M independently of one another stand for O and S;
n and r stand for 1 to 6 and m stands for 2 to 8;
P stands for a group selected from substituted or unsubstituted alkylene, preferably (CH₂)₁₋₆, or substituted or unsubstituted, aromatic or aliphatic N- or O-cycloalkylene; and
R independently of one another stands for a polymer group selected from chitosan and polylysine, wherein polylysine comprises poly-D-lysine and poly-L-lysine.

2. The implant according to Claim 1, wherein the coating containing the functionalised RGD-peptidomimetic RGD-P1 of formula (1) and/or RGD-P2 of formula (2) is formed as a cover layer.

3. The implant according to Claim 1 or 2, wherein the coating of the implant comprises a base layer and a cover layer applied to the base layer, said cover layer containing the functionalised RGD peptidomimetic RGD-P1 of formula (1) and/or RGD-P2 of formula (2).

4. The implant according to Claim 3, wherein the base layer comprises a layer that releases an active ingredient.

5. The implant according to Claim 4, wherein the active ingredient is paclitaxel, sirolimus and/or rapamycin, preferably the active ingredients sirolimus.

6. The implant according to one of Claims 1 to 5, wherein the implant is a stent, a sensor or an electrode.

7. The implant according to Claim 6, wherein the stent has a resorbable or permanent main body made of a metal or polymer material.

8. A method for producing an implant according to one of Claims 1 to 7, wherein the method comprises the following steps:
- coating a stent with a layer that contains or consists of chitosan and/or polylysine;
- covalently coupling a primary amine contained in chitosan and/or polylysine to the coupling group of a precursor of the functionalised RGD peptidomimetic RGD-P1 having formula (3): 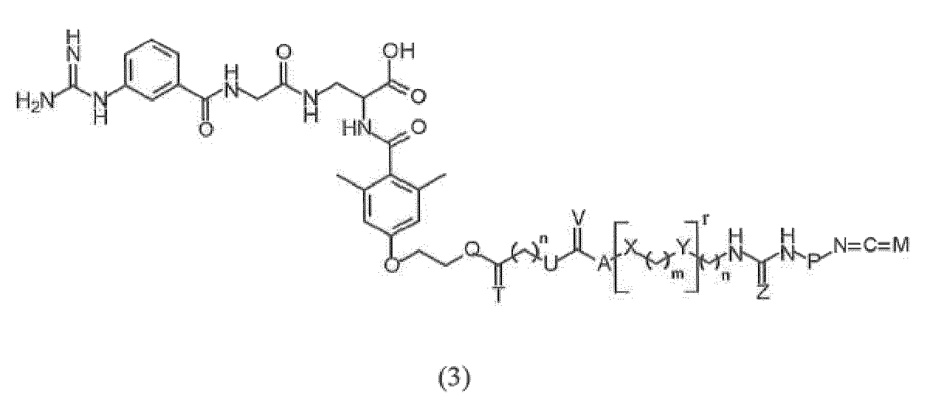 and/or
of a precursor of the functionalised RGD peptidomimetic RGD-P2 having formula (4): 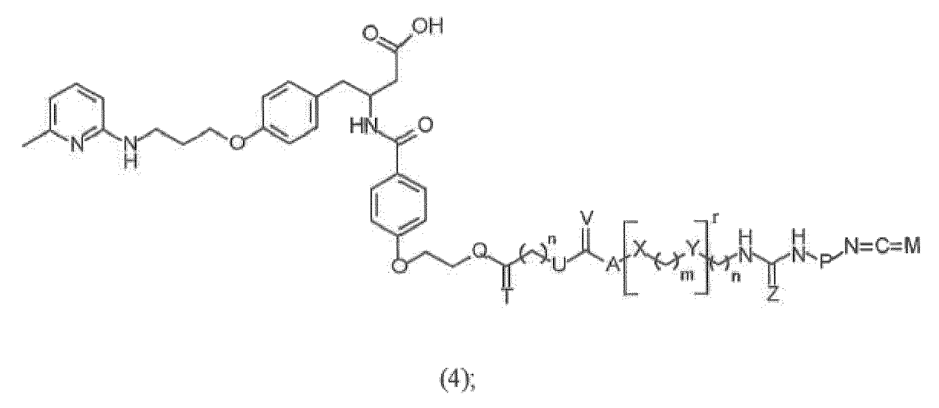 wherein
Q, T and U, V independently of one another stand for O or NH and Q#T and U#V;
A stands for O or (CH₂)₁₋₃ and X stands for CH₂, O, N or S, and when A is equal to O, X is CH₂;
Y stands for CH₂, O, N or S, and Z and M independently of one another stand for O or S;
n and r stand for 1 to 6 and m stands for 2 to 8;
P stands for a group selected from substituted or unsubstituted alkylene, preferably (CH₂)₁₋₆, or substituted or unsubstituted, aromatic or aliphatic N- or O-cycloalkylene.

9. A compound according to:
formula (1) 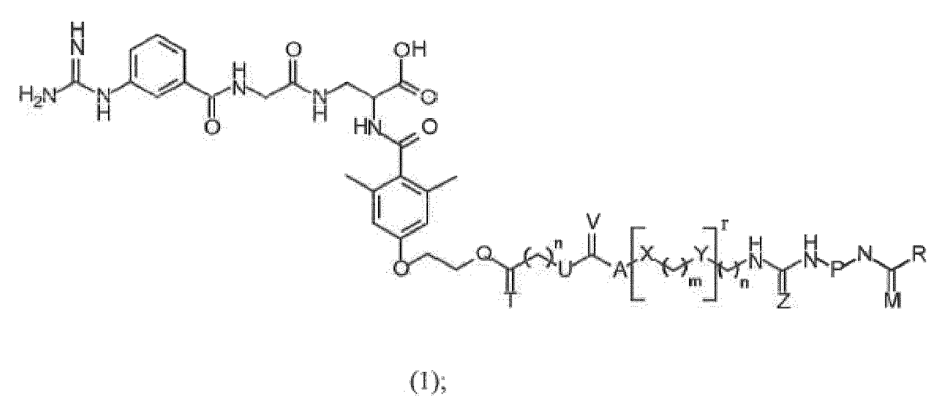
formula (2) 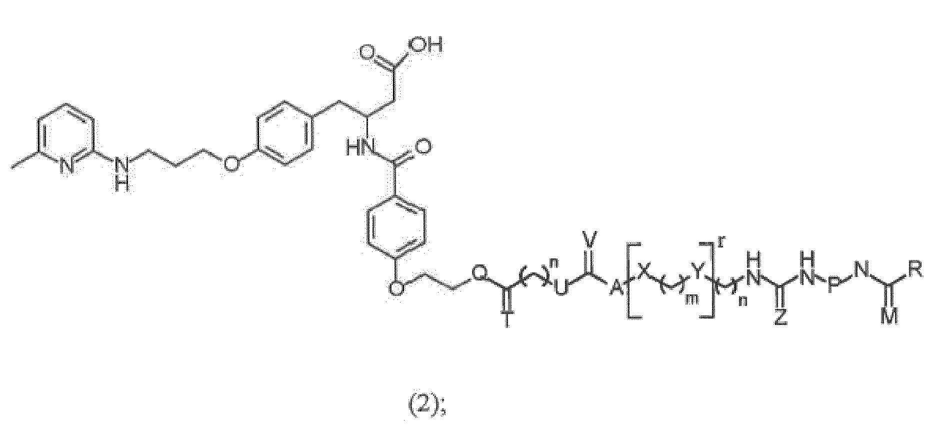
formula (3) 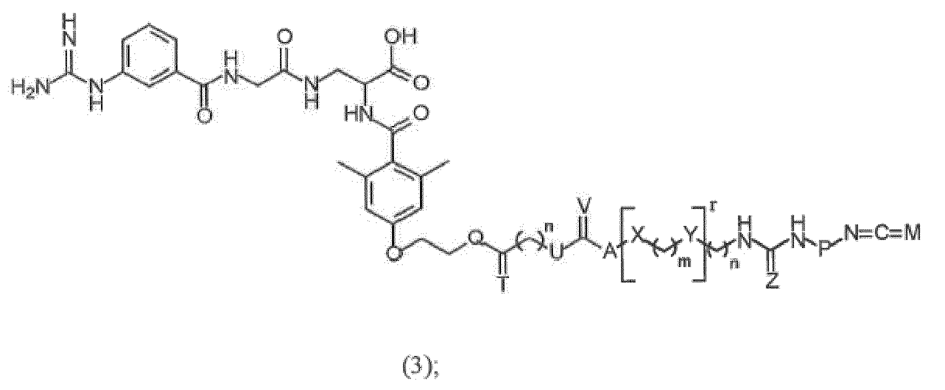 or
formula (4) 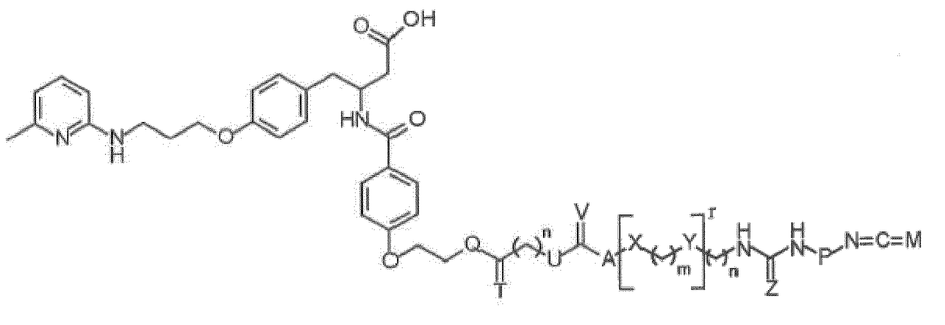 wherein
Q, T and U, V independently of one another stand for O or NH and Q≠T and U≠V;
A stands for O or (CH₂)₁₋₃ and X stands for CH₂, O, N or S, and when A is equal to O, X is CH₂;
Y stands for CH₂, O, N or S, and Z and M independently of one another stand for O or S;
n and r stand for 1 to 6 and m stands for 2 to 8;
P stands for a group selected from substituted or unsubstituted alkylene, preferably (CH₂)₁₋₆, or substituted or unsubstituted, aromatic or aliphatic N- or O-cycloalkylene; and
R independently for one another stands for a polymer group selected from chitosan and polylysine, wherein polylysine comprises poly-D-lysine and poly-L-lysine.

10. Use of a compound according to Claim 9 in a coating of an implant, in particular in the cover layer of stent that releases an active ingredient.

## Revendications

1. Implant comprenant un revêtement, le revêtement contenant, ou étant constitué par, un mimétique de peptides RGD fonctionnalisé RGD-P1 possédant la formule (1) : 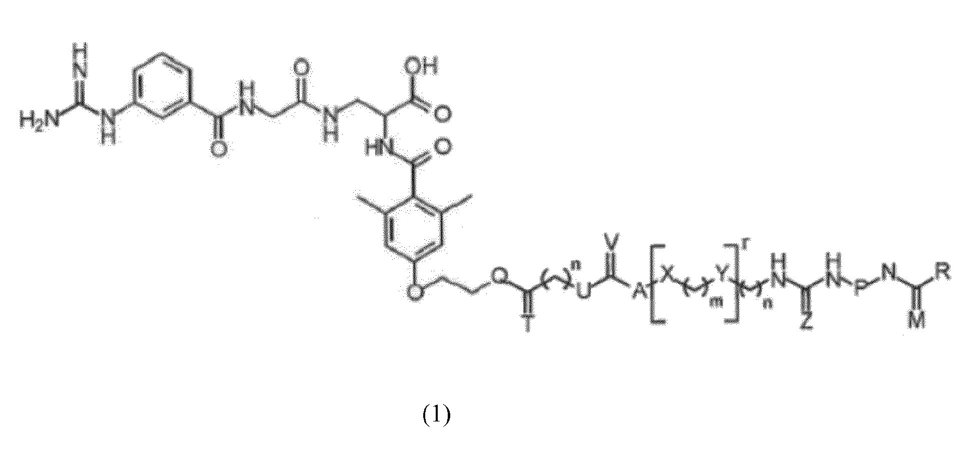 et/ou
un mimétique de peptides RGD fonctionnalisé RGD-P2 possédant la formule (2) : 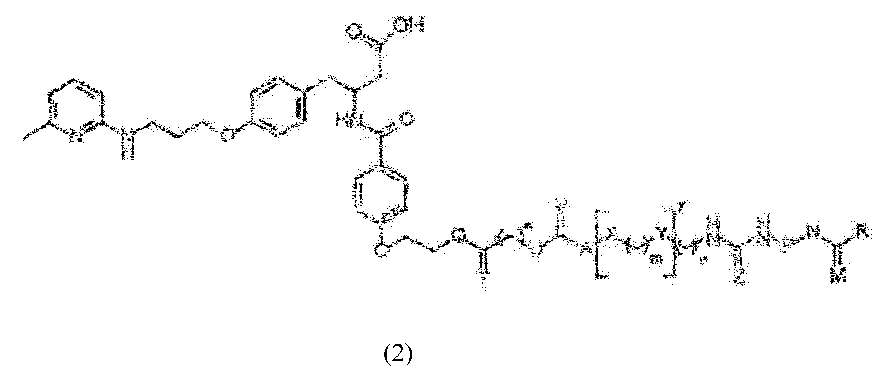 où
Q, T, ainsi que U, V, sont indépendamment les uns des autres un atome O ou un groupe NH, et Q ≠ T et U ≠ V ;
A est un atome O ou un groupe (CH₂)₂₋₃ et X est un groupe CH₂, un atome O, N ou S, et lorsque A est équivalent à l'atome O, X est un groupe CH₂ ;
Y est un groupe CH₂, un atome O, N ou S, ainsi que Z et M sont indépendamment les uns des autres des atomes O ou S ;
n et r sont des nombres entre 1 et 6 et m est un nombre entre 2 et 8 ;
P représente un groupe choisi parmi des groupes alkyles substitués ou non substitués, de préférence des groupes (CH₂)₁₋₆, ou des groupes N- ou O-cycloalkyles substitués ou non substitués, aromatiques ou aliphatiques, et
R est choisi indépendamment des autres parmi un groupe polymère choisi parmi le chitosan et la polylysine, la polylysine comprenant la poly-D-lysine et la poly-L-lysine.

2. Implant selon la revendication 1, dans lequel le revêtement contenant le mimétique de peptides RGD fonctionnalisé RGD-P1 de formule (1) et/ou RGD-P2 de formule (2) est conçu sous la forme d'une couche de recouvrement.

3. Implant selon les revendications 1 ou 2, dans lequel le revêtement de l'implant comprend une couche de base et une couche de recouvrement appliquée sur la couche de base ladite couche de recouvrement contenant le mimétique de peptides RGD fonctionnalisé RGD-P1 de formule (1) et/ou RGD-P2 de formule (2).

4. Implant selon la revendication 3, dans lequel la couche de base présente une couche libérant une substance active.

5. Implant selon la revendication 4, dans lequel la substance active est du Paclitaxel, du Sirolimus et/ou de la Rapamycine, le Sirolimus étant la substance active préférée.

6. Implant selon l'une des revendications 1 à 5, l'implant étant une endoprothèse, un capteur ou une électrode.

7. Implant selon la revendication 6, dans lequel l'endoprothèse présente un corps de base résorbable ou permanent constitué d'un matériau métallique ou polymère.

8. Procédé de fabrication d'un implant selon l'une des revendications 1 à 7, le procédé comprenant les étapes :
- de revêtement d'une endoprothèse avec une couche qui contient ou est constituée de chitosan et/ou de polylysine ;
- d'un couplage covalent d'une amine primaire contenant du chitosan et/ou de la polylysine avec le groupe de couplage d'un précurseur du mimétique de peptides RGD fonctionnalisé RGD-P1 possédant la formule (3) : 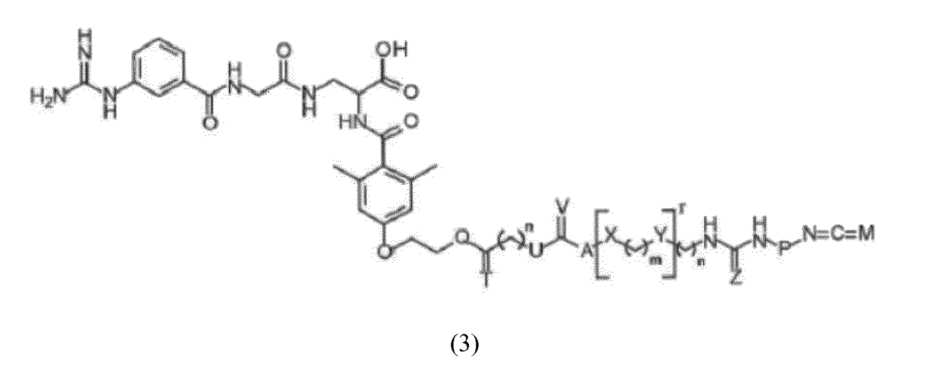 et/ou
d'un précurseur du mimétique de peptides RGD fonctionnalisé RGD-P2 possédant la formule (4) : 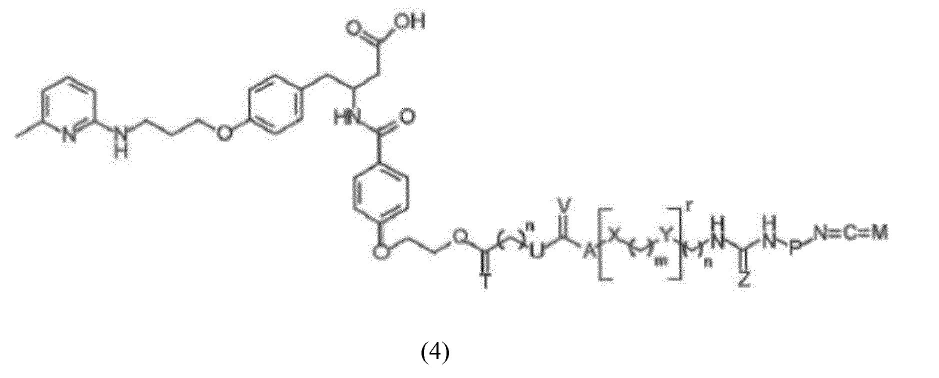 où
Q, T, ainsi que U, V, sont indépendamment les uns des autres un atome O ou un groupe NH, et Q ≠ T, et U ≠ V ;
A est un atome O ou un groupe (CH₂)₁₋₃ et X est un groupe CH₂, un atome O, N ou S et lorsque A est un atome O, X est un groupe CH₂ ;
Y est un groupe CH₂, un atome O, N ou S, ainsi que Z et M sont indépendamment les uns des autres des atomes O ou S ;
n et r sont des nombres de 1 à 6 et m est un nombre de 2 à 8 ;
P est un groupe choisi parmi des groupes alkyles substitués ou non substitués, de préférence des groupes (CH₂)₁₋₆ ou des groupes N- ou O-cycloalkyles substitués ou non substitués, aromatiques ou aliphatiques.

9. Composé selon :
la formule (1) 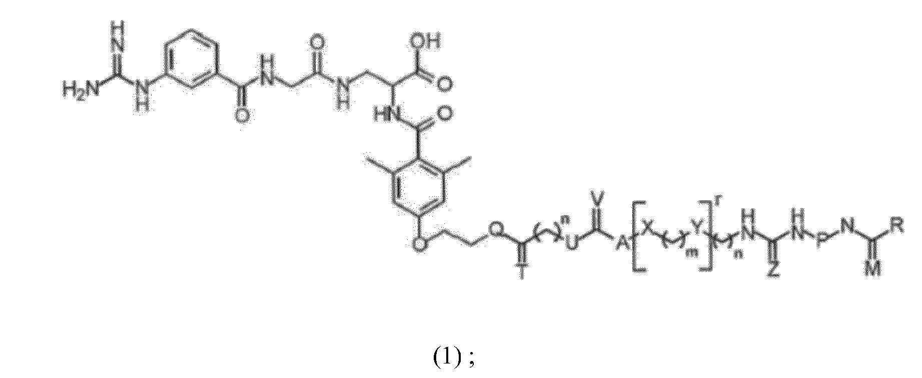
la formule (2) 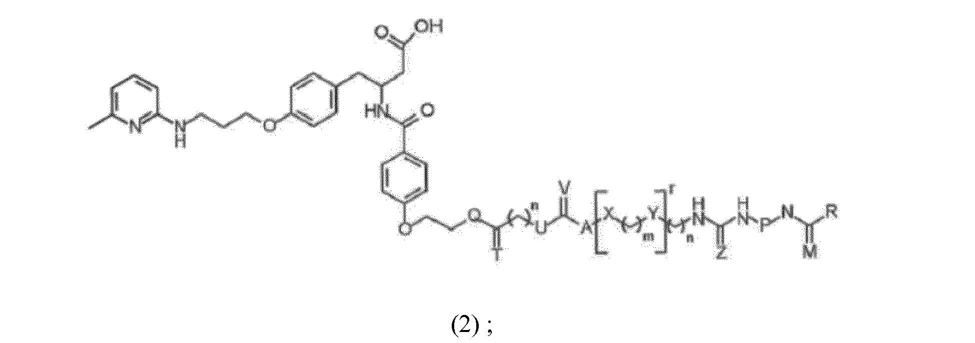
la formule (3) 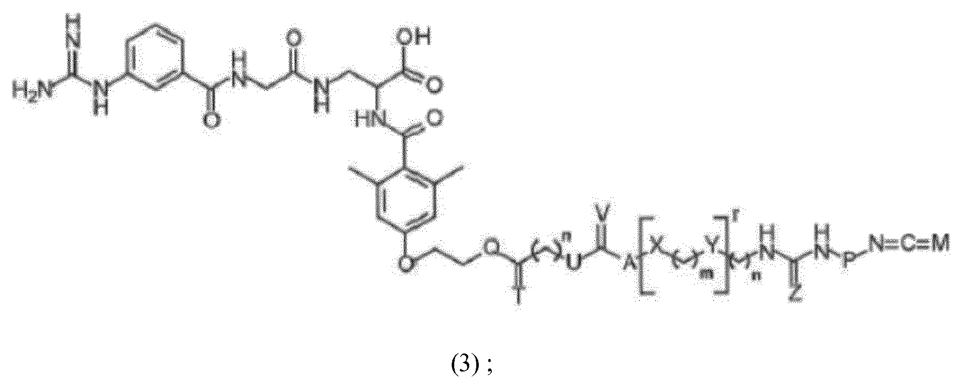 ou
la formule (4) 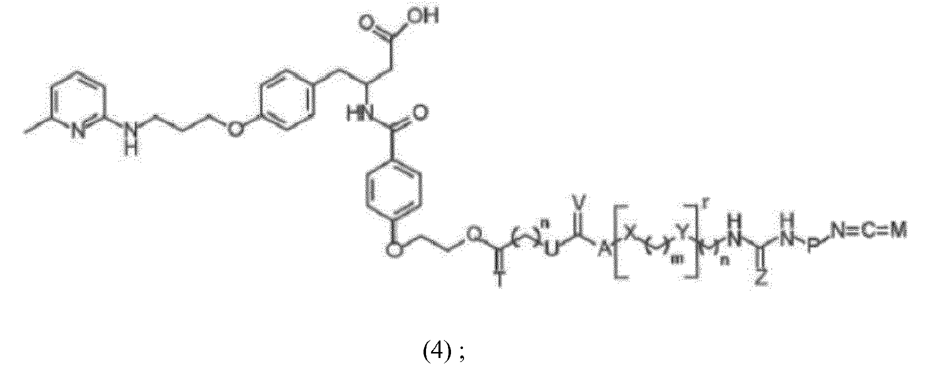 dans lesquelles
Q, T, ainsi que U, V, sont indépendamment les uns des autres un atome O ou un groupe NH, et Q ≠ T, et U ≠ V ;
A est un atome O ou un groupe (CH₂)₁₋₃ et X est un groupe CH₂, un atome O, N ou S et lorsque A est équivalent à l'atome O, X est un groupe CH₂ ;
Y est un groupe CH₂, un atome O, N ou S, ainsi que Z et M sont indépendamment les uns des autres des atomes O ou S,
n et r sont des nombres de 1 à 6 et m est un nombre de 2 à 8,
P est un groupe choisi parmi des groupes alkyles substitués ou non substitués, de préférence des groupes (CH₂)₁₋₆, ou des groupes N- ou O-cycloalkyles substitués ou non substitués, aromatiques ou aliphatiques ; et
R est indépendamment des autres un groupe de polymères choisis parmi le chitosan et la polylysine, la polylysine comprenant la poly-D-lysine et la poly-L-lysine.

10. Utilisation d'un composé selon la revendication 9 dans un revêtement d'un implant, notamment dans la couche de recouvrement d'une endoprothèse libérant une substance active.
